(19) Europäisches Patentamt
European Patent Office
Office européen des brevets

(11) Veröffentlichungsnummer: **0 067 981**
**A2**

# (12) EUROPÄISCHE PATENTANMELDUNG

(21) Anmeldenummer: 82104776.8

(22) Anmeldetag: 01.06.82

(51) Int. Cl.³: C 07 D 231/20
A 01 N 43/56

(30) Priorität: 10.06.81 JP 88062/81

(43) Veröffentlichungstag der Anmeldung:
29.12.82 Patentblatt 82/52

(84) Benannte Vertragsstaaten:
BE CH DE FR GB IT LI NL

(71) Anmelder: NIHON TOKUSHU NOYAKU SEIZO K.K.
No.4, 2-Chome, Nihonbashi Honcho
Chuo-ku Tokyo 103(JP)

(72) Erfinder: Aya, Masahiro
2-844, Suzuki-cho
Kodaira-shi Toyko(JP)

(72) Erfinder: Saito, Junichi
3-7-12, Osawa
Mitaka-shi Tokyo(JP)

(72) Erfinder: Kamochi, Atsumi
2-24-10, Higashitoyoda
Hino-shi Tokyo(JP)

(72) Erfinder: Moriya, Koichi
39-15, Namiki-cho
Hachioji-shi Tokyo(JP)

(74) Vertreter: Schumacher, Günter, Dr. et al,
c/o Bayer AG Zentralbereich Patente, Marken und Lizenzen
D-5090 Leverkusen 1 Bayerwerk(DE)

(54) **Neue Pyrazol-Derivate, Verfahren zu ihrer Herstellung und ihre Verwendung als Herbizide.**

(57) Neue Pyrazol-Derivate der allgemeinen Formel

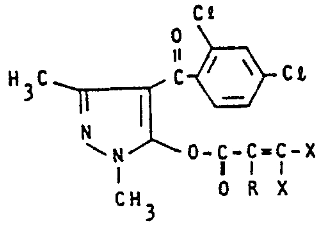

(1)

worin
X für Halogen-Atome und
R für ein Halogen-Atom oder eine Aryl-Gruppe, die gegebenenfalls durch Halogen oder ein $C_1$- bis $C_6$-Alkyl substituiert ist, stehen,
ein Verfahren zu ihrer Herstellung und ihre Verwendung als Herbizide.

EP 0 067 981 A2

Croydon Printing Company Ltd.

NIHON TOKUSHU NOYAKU SEIZO K.K., Tokyo, Japan

Bi/Bck
Ia

Neue Pyrazol-Derivate, Verfahren zu ihrer Herstellung und ihre Verwendung als Herbizide

Die vorliegende Erfindung betrifft bestimmte neue Pyrazol-Derivate, ein Verfahren zu ihrer Herstellung und ihre Verwendung als Herbizide.

In der vor dem Anmeldungsdatum der vorliegenden Anmeldung veröffentlichten japanischen Veröffentlichung Nr. Sho 54-36648 wird offenbart, daß die Verbindungen der allgemeinen Formel

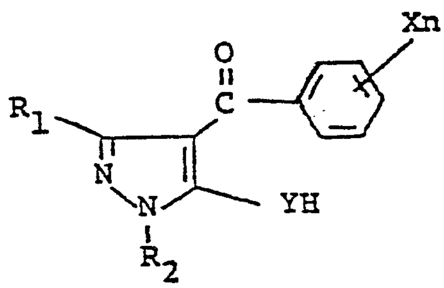

(A)

,

in der $R_1$ für ein Wasserstoff-Atom oder eine niedere Alkyl-Gruppe, $R_2$ für eine niedere Alkyl-Gruppe, und X für ein Halogen-Atom oder eine Nitro-, niedere Alkyl-, Trifluoromethyl-, niedere Alkoxy-, niedere Alkansulfonyl-, Cyano-, niedere Alkylthio- oder aliphatische Acyl-Gruppe stehen,
n 1, 2, 3 oder 4 ist und,
wenn n 2, 3 oder 4 ist, die Reste X gleich oder voneinander verschieden sein können und Y für ein Sauerstoff-Atom oder ein Schwefel-Atom steht, sowie deren Salze oder deren Ester mit einer organischen Säure ausgewählt aus aliphatischen, alicyclischen oder aromatischen Carbonsäuren, Carbaminsäuren, Sulfonsäuren, Thiophosphorsäure, Diestern, Kohlensäure- oder Thiokohlensäure-Monoestern, zweibasischen Säuren und Ester mit organischen Säuren der allgemeinen Formel

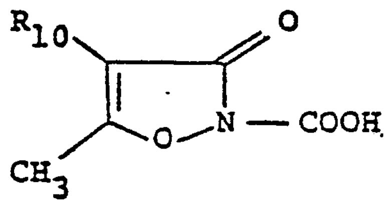

,

in der $R_{10}$ für ein Wasserstoff-Atom oder ein Halogen-Atom steht, herbizide Wirkung besitzen.

Die vorliegende Erfindung macht nunmehr als neue Verbindungen die Pyrazol-Derivate der allgemeinen Formel

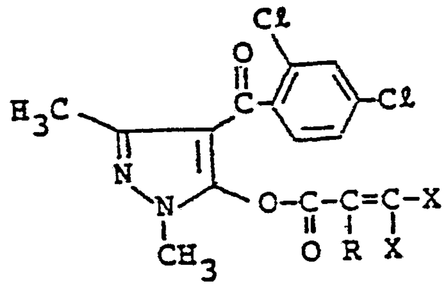

(I)

verfügbar, in der
X für Halogen-Atome und
R für ein Halogen-Atom oder eine Aryl-Gruppe, die gegebenenfalls durch Halogen oder ein $C_1$- bis $C_6$-Alkyl substituiert ist, stehen.

Die vorliegende Erfindung umfaßt auch ein Verfahren zur Herstellung eines Pyrazol-Derivats der Formel (I), bei dem 1,3-Dimethyl-4-(2,4-dichlorobenzoyl)-5-hydroxypyrazol mit einem Säure-Halogenid der allgemeinen Formel

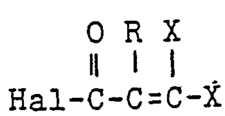

(II)

umgesetzt wird, in der
X und R die im vorstehenden angegebenen Bedeutungen haben und
Hal für ein Halogen-Atom steht.

Im Rahmen des erwähnten Standes der Technik werden die Verbindungen der vorliegenden Erfindung, die den

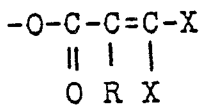

-Rest besitzen, nicht offenbart.

Es wurde gefunden, daß die neuen Pyrazol-Derivate der Formel (I) der vorliegenden Erfindung ausgezeichnete herbizide Wirksamkeit besitzen. Weiterhin zeigen die Verbindungen der vorliegenden Erfindung eine hervorragende herbizide Wirkung bereits bei niedriger Dosierung im Vergleich zu den nach dem Stand der Technik bekannten Verbindungen. Darüber hinaus wurde gefunden, daß die Verbindungen der vorliegenden Erfindung von Nutzpflanzen gut vertragen werden, so daß sie sicher anwendbar sind. Die vorliegende Erfindung verkörpert somit einen bedeutenden technischen Fortschritt.

Das Verfahren gemäß der vorliegenden Erfindung kann durch die folgende Gleichung dargestellt werden:

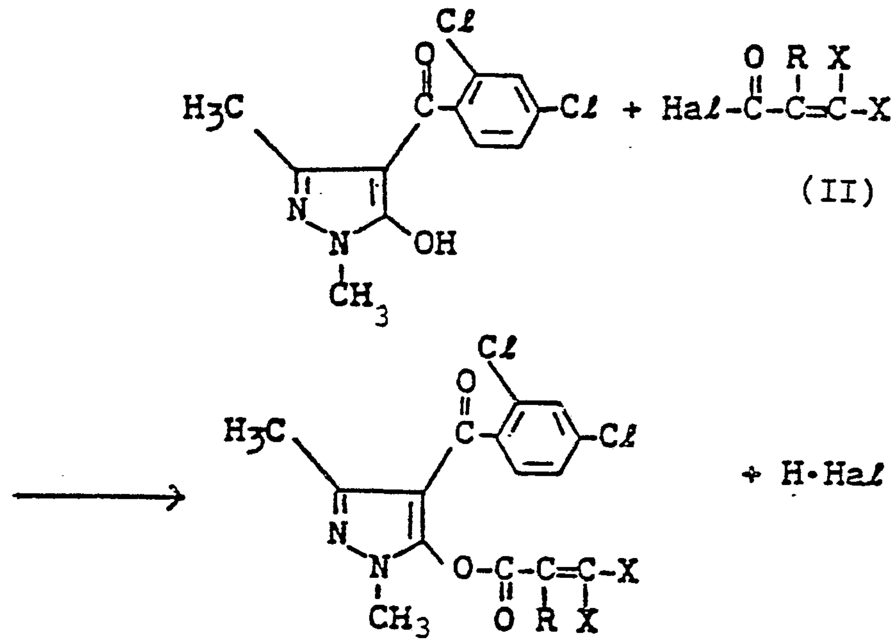

(II)

(I)

(worin X, R und Hal die im Vorstehenden angegebenen Bedeutungen haben).

Bevorzugte Verbindungen gemäß der vorliegenden Erfindung und bevorzugte Ausgangsmaterialien der Formel (II) sind solche, in denen X für Halogen-Atome wie Fluor-, Chlor-, Brom- oder Iod-Atome und R für Halogen-Atom wie die vorgenannten oder eine Aryl-Gruppe wie Phenyl oder Naphthyl, gegebenenfalls substituiert durch ein Halogen-Atom wie die vorgenannten oder eine $C_1$- bis $C_6$-Alkyl-Gruppe, wie Methyl, Ethyl, Propyl, Isopropyl oder n-, iso-, sec- oder tert-Butyl stehen.

Besonders bevorzugte Verbindungen und Ausgangsmaterialien sind diejenigen, in denen X für Chlor-Atome steht, und diejenigen, in denen R für ein Chlor-Atom oder Phenyl, Naphthyl, Monochloro-substituiertes Phenyl oder o-,m- oder p-Tolyl steht. Eine ganz besonders bevorzugte Verbindung ist die Verbindung, in der X und R beide für Chlor-Atome stehen.

Beispiele für bevorzugte Säurehalogenide der allgemeinen Formel (II) zum Einsatz als Ausgangsstoff bei dem Verfahren der vorliegenden Erfindung sind die folgenden: 3,3-Dichloro-2-phenyl-acrylsäurechlorid, 3,3-Dichloro-2-p-tolylacrylsäurechlorid, 3,3-Dichloro-2-m-tolylacrylsäurechlorid, 3,3-Dichloro-2-o-tolylacrylsäurechlorid, 3,3-Dichloro-2-p-chlorophenylacrylsäurechlorid, 3,3-Dichloro-2-α-naphthylacrylsäurechlorid, Trichloroacrylsäurechlorid und neben diesen die anderen entsprechenden Halogenide, vorzugsweise die Bromide.

Wenn 1,3-Dimethyl-4-(2,4-dichlorobenzoyl)-5-hydroxypyrazol und 3,3-Dichloro-2-phenylacrylsäurechlorid als Ausgangsstoffe verwendet werden, wird das Verfahren gemäß der vorliegenden Erfindung durch die folgende Gleichung erläutert

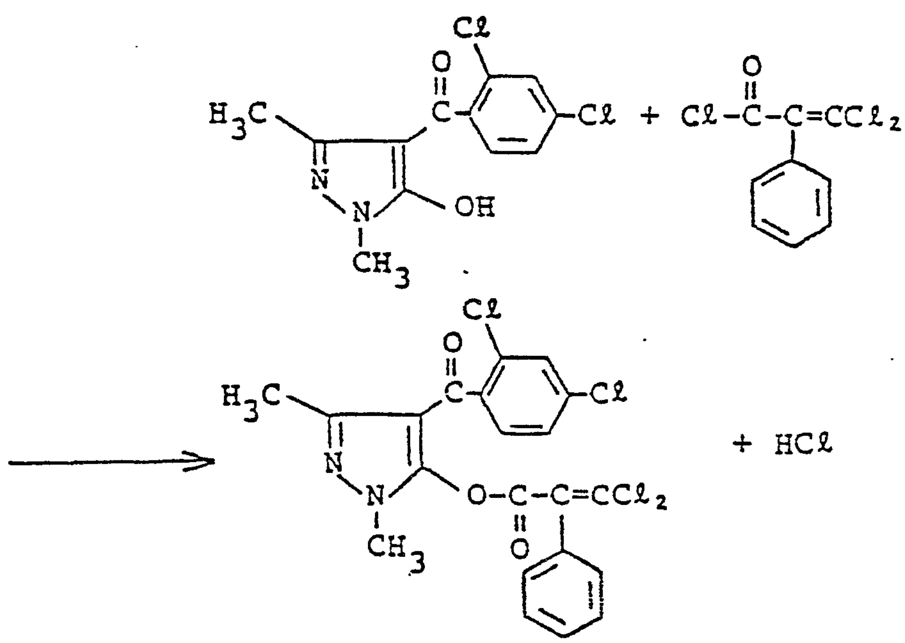

Das Verfahren gemäß der vorliegenden Erfindung wird vorzugsweise in Gegenwart eines Lösungsmittels oder Verdünnungsmittels durchgeführt. Für diesen Zweck können alle inerten Lösungsmittel und Verdünnungsmittel verwendet werden. Als Beispiele für geeignete Lösungsmittel oder Verdünnungsmittel zur Verwendung gemäß der vorliegenden Erfindung seien die folgenden erwähnt: Wasser, aliphatische, cycloaliphatische und aromatische Kohlenwasserstoffe, die in jedem Falle gegebenenfalls chloriert sein können, (beispielsweise Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol), Ether (beispielsweise Dimethylether, Methylethylether, Di-isopropylether, Dibutylether, Propylenoxid, Dioxan und Tetrahydrofuran), Ketone (beispielsweise Aceton, Methylethylketon, Methylisopropylketon und Methylisobutylketon), Nitrile (beispielsweise Acetonitril, Propionitril und Acrylnitril), Ester (beispielsweise Ethylacetat und Amylacetat), Säureamide (beispielsweise Dimethylformamid und Dimethylacetamid), Sulfone, Sulfoxide (beispielsweise Dimethylsulfoxid), Sulfolan sowie Basen (beispielsweise Pyridin).

Das Verfahren der vorliegenden Erfindung kann in Gegenwart eines Säure-bindenden Mittels wie eines Hydroxids eines Alkalimetalls, eines Carbonats, eines Hydrogencarbonats, eines Alkoholats, eines tertiären Amins (beispielsweise Triethylamin), Diethylanilin oder Pyridin durchgeführt werden.

Das Verfahren der vorliegenden Erfindung kann innerhalb eines weiten Temperaturbereichs durchgeführt werden, beispielsweise bei einer Temperatur zwischen -20°C und

dem Siedepunkt der Reaktionsmischung, und vorzugsweise bei einer Temperatur zwischen 0°C und 100°C. Die Reaktion wird vorzugsweise bei atmosphärischem Druck durchgeführt, kann jedoch auch unter vermindertem oder erhöhtem Druck durchgeführt werden.

Die wirksamen Verbindungen gemäß der Erfindung beeinflussen das Pflanzenwachstum und können deshalb als Entlaubungsmittel, Abbrennmittel, Mittel zur Zerstörung breitblättriger Pflanzen, die Keimung hemmende Mittel und insbesondere als Unkrautvernichtungsmittel verwendet werden. Unter "Unkräutern" im weitesten Sinne werden Pflanzen verstanden, die an Stellen wachsen, an denen sie nicht erwünscht sind.

Ob die Verbindungen gemäß der Erfindung als total wirksame Herbizide oder selektiv wirksame Herbizide wirken, hängt im wesentlichen von der verwendeten Menge ab.

Die Wirkstoffe gemäß der vorliegenden Erfindung können beispielsweise zur Bekämpfung der nachstehenden Pflanzen verwendet werden:

Dicotyledonen-Unkräuter der Genera Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum,Sesbania, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum; und

Monocotyledonen-Unkräuter der Genera Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis,

Sagittaria, Eleocharis, Scirpus, Paspalum, Ischaemum, Sphenoclea, Dactyloctenium, Agrostis, Alopecurus und Apera.

Die Wirkstoffe gemäß der vorliegenden Erfindung können beispielsweise als selektive Herbizide in den folgenden Kulturen verwendet werden:

Dicotyledonen-Kulturen der Genera Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucubirta; und

Monocotyledonen-Kulturen der Genera Oryza, Zea, Triticum, Hordeum, Avena, Secale, Sorghum, Panicum, Saccharum, Ananas, Asparagus und Allium.

Die Verwendung der Wirkstoffe gemäß der Erfindung ist jedoch in keiner Weise auf diese Genera beschränkt, sondern sie schließt auch andere Pflanzen in der gleichen Weise ein.

Je nach den Konzentrationen können die Verbindungen für die totale Unkrautbekämpfung, beispielsweise auf Industrie-Gelände und Bahnkörpern und auf Wegen und Plätzen mit oder ohne Baumbestand verwendet werden. Ebenso können die Verbindungen zur Unkrautbekämpfung in perennierenden Kulturen, beispielsweise Aufforstungen, Zierbaumpflanzungen, Obstgärten, Weinbergen und -gärten, Zitruswäldern, Nußbaumpflanzungen, Bananenplantagen, Kaffeeplantagen, Teeplantagen, Gummiplantagen, Ölpalmenplantagen, Kakaoplantagen, Weichfruchtanplanzungen und Hopfenfeldern sowie zur selektiven Unkrautbekämpfung in einjährigen Kulturen verwendet werden.

Es ist darauf hinzuweisen, daß die Verbindungen gemäß der vorliegenden Erfindung eine ausgezeichnete Selektivität gegenüber Reispflanzen unter Überflutungsbedingungen zeigen. Die Verbindungen der vorliegenden Erfindung sind zur Bekämpfung einer breiten Vielzahl von Reisfeld-Unkräutern wirksam, beispielsweise:

die Dicotyledonen-Unkräuter Rotala indica, Lindernia pyxidaria, Ludwiga prostrata, Potamogeton distinctus und Elatine triandra; und die die Monocotyledonen-Unkräuter Echinochloa crus-galli, Monochoria vaginalis, Eleocharis acicularis, Eleocharis Kuroguwai, Cyperus serotinus, Sagittaria pygmaea, Alisma canaliculatum und Scirpus juncoides.

Weiterhin zeigen die Verbindungen gemäß der vorliegenden Erfindung auch eine besondere herbizide Wirkung zur Bekämpfung der folgenden Unkräuter:

die Dicotyledonen-Unkräuter Polygonum sp., Chenopodium album, Stellaria media und Portulaca oleracea; und die Monocotyledonen-Unkräuter Echinochloa crus-galli, Digitaria adscendens und Cyperus iria.

Die Wirkstoffe können in die üblichen Formulierungen umgewandelt werden, wie Lösungen, Emulsionen, Suspensionen, Pulver, Stäubemittel, Schäume, Pasten, lösliche Pulver, Granulate, Aerosole, Suspensions-Emulsionskonzentrate, natürliche und synthetische, mit dem Wirkstoff imprägnierte Materialien, sehr feine Kapseln aus polymeren Substanzen,

sowie Formulierungen zur ULV (ultra-low-volume)-Kalt- und Warmvernebelung.

Diese Präparate können in bekannter Weise hergestellt werden, beispielsweise durch Mischen der Wirksubstanzen mit Streckmitteln, d.h. flüssigen oder festen Verdünnungsmitteln oder Trägern, gegebenenfalls unter Verwendung von obeflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, alipahtische oder alicyclische Kohlenwasserstoffe wie Cyclohexan oder Paraffine, beispielsweise Mineralölfraktionen, Alkohole wie Buthanol oder Glycol sowie deren Ether und Ester, Ketone wie Aceton, Methylethylketon, Methylisobutylketon oder Cyclohexanon, oder stark polare Lösungsmittel wie Dimethylformamid und Dimethylsulfoxid,sowie Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silicate,verwendet werden. Als feste Träger für Granulat können zerkleinerte und fraktionierte natürliche Gesteine wie Calcit, Marmor, Bimsstein, Sepiolith und Dolomit sowie synthetisches Granulat aus anorganischen und organische Mehlen sowie Granulat aus organischem Material wie Sägemehl, Kokusnußschalen, Maiskolben und Tabakstengeln verwendet werden.

Als Emulgatoren und/oder Schaumbildner können nichtionische und anionische Emulgatoren wie Polyoxyethylen-Fettsäureester, Polyoxyethylen-Fettalkoholether, beispielsweise Alkylaryl-Polyglycolether, Alkylsulfonate, Alkylsulfate, Arylsulfonate sowie Hydrolyseprodukte des Albumins verwendet werden. Zu Dispergiermitteln gehören beispielsweise Ligninsulfit-Ablaugen und Methylcellulose.

Haftmittel wie Carboxymethylcellulose und natürliche und synthetische Polymerisate in Form von Pulver, Granulat oder Latices, wie Gummiarabicum, Polyvinylalkohol und Polyvinylacetat können in den Präparaten verwendet werden.

Es ist möglich, Färbemittel wie anorganische Pigmente, beispielsweise Eisenoxid, Titanoxid und Preußisch Blau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink zu verwenden.

Die Präparate enthalten im allgemeinen 0,001 bis 100 Gew.-%, vorzugsweise von 0,05 bis 95 Gew.-% der Wirksubstanz .

Die Wirkstoffe gemäß der Erfindung können in den Formulierungen oder in den verschiedenen Anwendungsformen im Gemisch mit anderen wirksamen Verbindungen, wie Fungiziden, Bakteriziden, Insektiziden, Nematiziden, Herbiziden, vogelabweisenden Mitteln, Wachstumsfaktoren, Pflanzennährstoffen und Mitteln zur Verbesserung der Bodenstruktur, vorhanden sein.

Die Wirkstoffe können als solche, in Form ihrer Präparate oder als daraus hergestellte Gebrauchsformen verwendet werden, z.B. als gebrauchsfertige Lösungen, Emulsionen, Suspensionen, Pulver, Pasten und Granulate. Sie können in üblicher Weise, beispielsweise durch Sprühen, Verstäuben, Stäuben, Streuen und Bewässern angewandt werden.

Es ist auch möglich, die Wirksubstanzen mit Hilfe des ULV-Verfahrens (ultra-low-volume method) anzuwenden, wodurch es möglich ist, die Verbindungen in einer Konzentration bis zu 100% einzusetzen. Die Wirksubstanzen können auch in den Boden eingearbeitet werden.

Im praktischen Gebrauch beträgt der Gehalt an Wirkstoffen in den genannten verschiedenartigen Formulierungen oder gebrauchsfertigen Präparaten im allgemeinen 0,01 bis 95 Gew.-%, vorzugsweise 0,05 bis 60 Gew.-%.

Die aufzuwendende Menge des Wirkstoffs kann innerhalb eines beträchtlichen Bereichs variiert werden. Sie hängt im wesentlichen von der Art der gewünschten Wirkung ab. Im allgemeinen werden Mengen des Wirkstoffs zwischen 0,1 und 2,0 kg/ha, vorzugsweise zwischen 0,25 und 2 kg/ha, eingesetzt.

Die vorliegende Erfindung umfaßt auch herbizide Zusammensetzungen, die als Wirkstoff eine Verbindung gemäß der vorliegenden Erfindung in Mischung mit einem festen oder aus einem Flüssiggas bestehenden Verdünnungsmittel oder Träger oder in Mischung mit einem flüssigen, ein oberflächenaktives Mittel enthaltenden Verdünnungsmittel

oder Träger enthalten.

Die vorliegende Erfindung umfaßt weiterhin ein Verfahren zur Unkrautbekämpfung, das dadurch gekennzeichnet ist, daß man auf die Unkräuter oder ihren Lebensraum eine Verbindung gemäß der vorliegenden Erfindung allein oder in Form einer Zusammensetzung, die als Wirkstoff eine Verbindung der vorliegenden Erfindung in Mischung mit einem Verdünnungsmittel oder Träger enthält, aufbringt.

Die vorliegende Erfindung ist ferner auf Nutzpflanzen gerichtet, die gegen Schäden durch Unkräuter dadurch geschützt sind, daß sie auf Flächen angebaut werden, auf denen unmittelbar vor und/oder während der Zeit des Anbaues eine Verbindung gemäß der vorliegenden Erfindung allein oder in Mischung mit einem Verdünnungsmittel oder Träger aufgebracht wurden.

Es wird ersichtlich, daß die üblichen Methoden der Erzeugung von Nutzpflanzenernten durch die vorliegende Erfindung verbessert werden können.

Die Zusammensetzungen gemäß der Erfindung und die herbizide Wirksamkeit der Verbindungen der vorliegenden Erfindung werden durch die folgenden Beispiele veranschaulicht. In diesen Beispielen sind die Verbindungen gemäß der Erfindung jeweils durch die (in Klammern genannte) Nummer des entsprechenden Herstellungsbeispiels bezeichnet. Die Angabe von Teilen und Verhältnissen beziehen sich auf das Gewicht.

Die bekannten Vergleichsverbindungen sind folgendermaßen definiert:

Nit 143

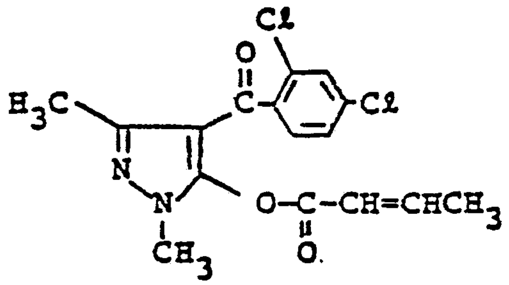

A-1

(eine Verbindung, die in der JP-AS 36648/79 offenbart wurde);

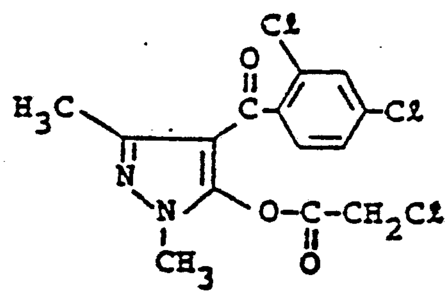

A-2

(eine Verbindung, die in der JP-AS 36648/79 offenbart wurde).

Beispiel i

Ein benetzbares Pulver wurde hergestellt durch Mahlen und Mischen von 15 Teilen der Verbindung (1), 80 Teilen einer aus pulvriger Diatomeenerde und pulvrigem Ton (1:5) bestehenden Mischung, 2 Teilen Natrium-Alkylbenzolsulfonat und 3 Teilen eines Kondensats aus Natrium-Alkylnaphthalinsulfonat und Formalin hergestellt. Dieses wurde vor dem Sprühen mit Wasser verdünnt.

Beispiel ii

Eine Emulsion wurde durch Vermischen und Rühren von 15 Teilen der Verbindung (2), 70 Teilen Xylol, 8 Teilen Polyoxyethylen-Alkylphenylether und 7 Teilen Kalzium-Alkylbenzolsulfonat hergestellt. Diese wurde vor dem Sprühen mit Wasser verdünnt.

Beispiel iii

Ein Pulver zur Anwendung als Stäubemittel wurde durch Mahlen und Vermischen von 2 Teilen der Verbindung (3) und 98 Teilen Tonpulver hergestellt.

Beispiel iv

Ein Pulver zur Anwendung als Stäubemittel wurde durch Mahlen und Vermischen von 1,5 Teilen der Verbindung (4), 0,5 Teilen Isopropylhydrogenphosphat (PAP) und 98 Teilen Tonpulver hergestellt.

Beispiel v

Ein Granulat wurde hergestellt durch Hinzufügen von 25 Teilen Wasser zu einer aus 10 Teilen der Verbindung (5), 30 Teilen Bentonit (Montmorillonit), 58 Teilen Talkum und 2 Teilen Ligninsulfonat bestehenden Mischung, Kneten und Granulieren mittels eines Extruder-Granulators, wodurch ein Granulat mit einer Korngröße von 0,43 bis 2,00 mm (10 bis 40 mesh) erhalten wurde, das bei 40°C bis 50°C getrocknet wurde. Das erhaltene Granulat wurde durch Streuen aufgebracht.

Beispiel vi

Ein Granulat wurde hergestellt durch Beschicken eines Drehmischers mit 75 Teilen eines mineralischen Granulats mit einer Teilchengröße-Verteilung von 0,2 bis 2 mm Aufsprühen von 7 Teilen der Verbindung (7) gelöst in einem

organischen Lösungsmittel auf das Granulat unter Drehen des Mischers, so daß das Granulat homogen befeuchtet wurde und Trocknen bei 40°C bis 50°C. Das erhaltene Granulat wurde durch Streuen aufgebracht.

Beispiel A

Behandlung zur Bekämfpung von Reisfeld-Unkräutern unter Überflutungsbedinungen (Pot Test)

Herstellung einer Wirkstoff-Zusammensetzung

Lösungsmittel: 5 Gewichtsteile Aceton
Emulgator: 1 Gewichtsteil Benzyloxy-Polyglycolether.

Eine Wirkstoffzubereitung in Form eines emulgierbaren Konzentrats wurde durch Vermischen eines Gewichtsteils des Wirkstoffs mit den vorbezeichneten Mengen des Lösungsmittels und des Emulgators erhalten. Eine vorher bestimmte Menge des Präparats wurde durch Verdünnen mit Wasser erhalten.

Testverfahren

Wagner-Töpfe (2 $dm^2$) wurden mit Reisfeldboden gefüllt und dann bewässert. Zwei Reis-Setzlinge (Varietät: Kinmaze) im 2-3-Blattstadium (Pflanzen von etwa 10 cm Höhe) wurden in jeden Topf umgeplanzt. Nach dem Umpflanzen wurden Samenkörner von Reis, Echinochloa crus-galli, Monochoria vaginalis, Scirpus juncoides, Cyperus difformis und breitblättrigen Unkräutern eingesät, und Rhizome von Eleocharis acicularis sowie Knollen von Sagittaria pygmaea wurden eingepflanzt. Als Echinochloa crus-galli etwa bis zum 2-Blattstadium gewachsen war (etwa 7 bis 9 Tage nach der Einsaat), wurden die Töpfe mit Wasser bis

zu einer Tiefe etwa 6 cm gefüllt, und eine vorher festgelegte Menge des Wirkstoffs in Form einer Emulsion wurde dem Wasser mittels einer Pipette zugesetzt. Das Wasser in den Töpfen wurde im Laufe von 2 Tagen nach der Behandlung mit einer Geschwindigkeit von 2 cm/Tag heraussickern gelassen. 4 Wochen nach der Behandlung wurden die herbizide Wirkung und der Grad der Phytotoxizität mit Hilfe einer Skala von 0 bis 10 nach dem folgenden Maßstab bewertet.

Die herbizide Wirkung wurde im Vergleich zu unbehandelten Kontrollen wie folgt bewertet:

| Bewertung | Unkrautvernichtungsrate (bezogen auf die Kontrolle) |
|---|---|
| 10 : | 100% (verdorrt) |
| 9 : | mindestens 90%, jedoch weniger als 100% |
| 8 : | mindestens 80%, jedoch weniger als 90% |
| 7 : | mindestens 70%, jedoch weniger als 80% |
| 6 : | mindestens 60%, jedoch weniger als 70% |
| 5 : | mindestens 50%, jedoch weniger als 60% |
| 4 : | mindestens 40%, jedoch weniger als 50% |
| 3 : | mindestens 30%, jedoch weniger als 40% |
| 2 : | mindestens 20%, jedoch weniger als 30% |
| 1 : | mindestens 10%, jedoch weniger als 20% |
| 0 : | weniger als 10% (unwirksam) |

Die Phytotoxizität gegenüber den Nutzpflanzen wurde im Vergleich zu den unbehandelten Kontrollen wie folgt bewertet:

| Bewertung | Phytotoxizitätsrate im Vergleich zu den Kontrollen |
|---|---|
| 10 : | mindestens 90% (Ausrottung) |
| 9 : | mindestens 80%, jedoch weniger als 90% |
| 8 : | mindestens 70%, jedoch weniger als 80% |
| 7 : | mindestens 60%, jedoch weniger als 70% |
| 6 : | mindestens 50%, jedoch weniger als 60% |
| 5 : | mindestens 40%, jedoch weniger als 50% |
| 4 : | mindestens 30%, jedoch weniger als 40% |
| 3 : | mindestens 20%, jedoch weniger als 30% |
| 2 : | mindestens 10%, jedoch weniger als 20% |
| 1 : | mehr als 0, jedoch weniger als 10% |
| 0 : | 0% (keine Phytotoxizität) |

Die Testergebnisse sind in Tabelle 1 aufgeführt, in der die Symbole a bis g die folgenden Unkräuter bezeichnen:

a: Echinochloa crus-galli
b: Eleocharis acicularis
c: Cyperus difformis
d: Scirpus juncoides
e: Monochoria vaginalis
f: Sagittaria pygmaea

g: breitblättrige Unkräuter (Lindernia pyxidaria, Rotala indica, Elatine triandra)

Tabelle 1

| Verbindung No. | Wirkstoff-Dosis kg/ha | Herbizide Wirkung | | | | | | | Schadwirkung | |
|---|---|---|---|---|---|---|---|---|---|---|
| | | Unkraut | | | | | | | Nutzpflanze* | |
| | | a | b | c | d | e | f | g | r-1 | r-2 |
| 1 | 1.0<br>0.5<br>0.25 | 10<br>10<br>9 | 10<br>10<br>9 | 10<br>10<br>10 | 10<br>10<br>9 | 10<br>10<br>10 | 10<br>10<br>10 | 10<br>10<br>9 | 0<br>0<br>0 | 0<br>0<br>0 |
| 2 | 1.0<br>0.5<br>0.25 | 10<br>9<br>9 | 10<br>10<br>10 | 10<br>10<br>10 | 10<br>10<br>10 | 10<br>10<br>10 | 10<br>10<br>10 | 10<br>10<br>9 | 0<br>0<br>0 | 0<br>0<br>0 |
| 3 | 1.0<br>0.5<br>0.25 | 10<br>10<br>9 | 10<br>10<br>9 | 10<br>10<br>10 | 10<br>10<br>9 | 10<br>10<br>10 | 10<br>10<br>10 | 10<br>10<br>10 | 0<br>0<br>0 | 0<br>0<br>0 |
| 4 | 1.0<br>0.5<br>0.25 | 10<br>9<br>9 | 10<br>10<br>9 | 10<br>10<br>10 | 10<br>10<br>9 | 10<br>10<br>10 | 10<br>10<br>9 | 10<br>10<br>9 | 0<br>0<br>0 | 0<br>0<br>0 |
| 5 | 1.0<br>0.5<br>0.25 | 10<br>9<br>9 | 10<br>10<br>9 | 10<br>10<br>10 | 10<br>10<br>10 | 10<br>10<br>10 | 10<br>10<br>10 | 10<br>10<br>9 | 0<br>0<br>0 | 0<br>0<br>0 |
| 6 | 1.0<br>0.5<br>0.25 | 10<br>9<br>8 | 10<br>9<br>9 | 10<br>10<br>10 | 10<br>9<br>9 | 10<br>9<br>8 | 10<br>9<br>9 | 10<br>10<br>9 | 0<br>0<br>0 | 0<br>0<br>0 |
| 7 | 1.0<br>0.5<br>0.25 | 10<br>9<br>9 | 10<br>9<br>9 | 10<br>10<br>10 | 10<br>9<br>8 | 10<br>10<br>9 | 10<br>10<br>9 | 10<br>10<br>9 | 0<br>0<br>0 | 0<br>0<br>0 |
| Vergleich A - 1 | 1.0<br>0.5<br>0.25 | 8<br>5<br>2 | 5<br>1<br>0 | 8<br>5<br>2 | 8<br>5<br>2 | 8<br>5<br>2 | 6<br>3<br>1 | 8<br>4<br>2 | 0<br>0<br>0 | 0<br>0<br>0 |
| Vergleich A - 2 | 1.0<br>0.5<br>0.25 | 8<br>3<br>1 | 5<br>1<br>0 | 7<br>2<br>1 | 7<br>2<br>1 | 8<br>4<br>1 | 5<br>1<br>0 | 8<br>4<br>1 | 0<br>0<br>0 | 0<br>0<br>0 |

* Die Symbole in der Spalte "Nutzpflanze" bedeuten:
r-1 = umgepflanzte Reispflanze
r-2 = wachsende Reispflanze

Die folgenden Herstellungsbeispiele erläutern die Verfahren zur Herstellung der Verbindungen der vorliegenden Erfindung.

Herstellungsbeispiel 1

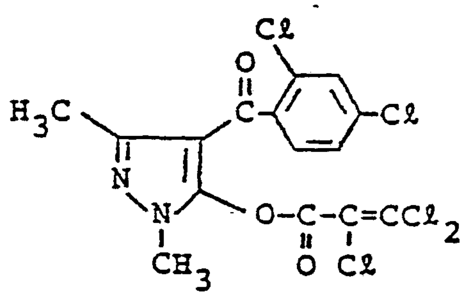

(1)

2,85 g 1,3-Dimethyl-4-(2,4-dichlorobenzoyl)-5-hydroxypyrazol und 1,01 g Triethylamin wurden in 20 ml Benzol aufgelöst. 1,94 g Trichloroacrylsäurechlorid wurden in die Lösung eingetropft. Die Reaktionsmischung wurde 3 h bei der Rückflußtemperatur des Benzols gerührt. Nach Beendigung der Reaktion wurde Wasser zu der Reaktionsmischung hinzugegeben, und die organische Schicht wurde abgetrennt und getrocknet. Das Benzol wurde unter vermindertem Druck abdestilliert, wonach 3,3 g 1,3-Dimethyl-4-(2,4-dichlorobenzoyl)-5-trichloroacryloyloxypyrazol in Form farbloser Kristalle vom Schmp.141-142°C erhalten wurden.

Die Verbindungen der vorliegenden Erfindung, die in Tabelle 2 aufgeführt sind, wurden in analoger Weise wie vorher beschrieben hergestellt.

Tabelle 2

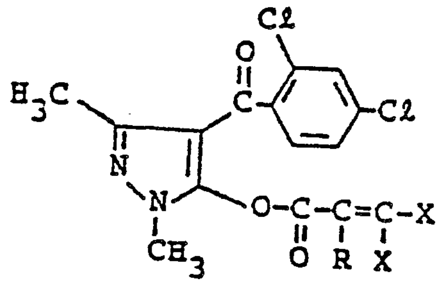

| Beispiel Nr. | X | R | Schmp. °C |
| --- | --- | --- | --- |
| 2 | Cl | 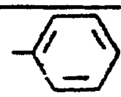 | 136 - 138.5 |
| 3 | Cl | 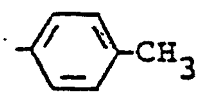 | 119 - 120 |
| 4 | Cl | 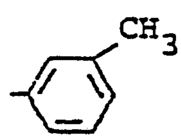 | 118 - 119 |
| 5 | Cl | 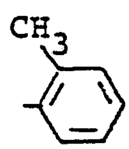 | 107 - 108 |
| 6 | Cl | 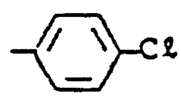 | 152 - 154 |
| 7 | Cl | 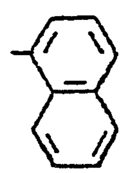 | 103 - 106 |

Patentansprüche

1. Pyrazol-Derivate der allgemeinen Formel

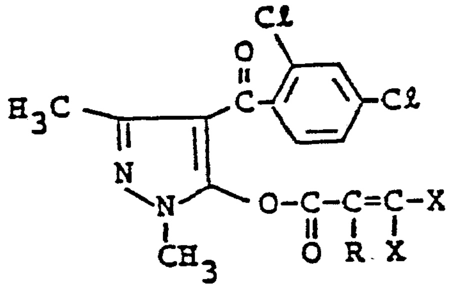

(I) ,

in der X für Halogen-Atome und R für ein Halogen-Atom oder eine Aryl-Gruppe, die gegebenenfalls durch Halogen oder $C_1$-bis $C_6$-Alkyl substitutuiert ist, stehen.

2. Verfahren zur Herstellung von Pyrazol-Derivaten der Formel (I) gemäß Anspruch 1, dadurch gekennzeichnet, daß man 1,3-Dimethyl-4-(2,4-dichlorbenzoyl)-5-hydroxy--pyrazol mit einem Säurehalogenid der allgemeinen Formel

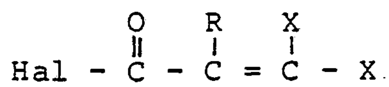

(II)

in welcher X und R die in Anspruch 1 genannte Bedeutung haben und Hal für Halogen steht, gegebenenfalls in Gegenwart eines inerten Lösungsmittels oder Verdünnungsmittels und gegebenenfalls in Gegenwart eines säurebindenden Mittels umsetzt.

3. Herbizide Mittel, gekennzeichnet durch einen Gehalt an mindestens einem Pyrazol-Derivat der Formel (I) gemäß Anspruch 1.

4. Verfahren zur Bekämpfung von Unkräutern, dadurch gekennzeichnet, daß man Pyrazol-Derivate der Formel (I) gemäß Anspruch 1 auf die Unkräuter oder ihren Lebensraum einwirken läßt.

5. Verwendung von Pyrazol-Derivaten der Formel (I) gemäß Anspruch 1 zur Bekämpfung von Unkräutern.

6. Verfahren zur Herstellung von herbiziden Mitteln, dadurch gekennzeichnet, daß man Pyrazol-Derivate der Formel (I) gemäß Anspruch 1 mit Streckmitteln und/oder oberflächenaktiven Mitteln vermischt.